# EUROPEAN PATENT APPLICATION

(11) **EP 2 128 624 A2**
(43) Date of publication of application: **02.12.2009**
(21) Application number: 09166316.1
(22) Date of filing: 09.03.2001
(51) Int. Cl.: G01N 33/58

(54) **Ultrasensitive non-isotopic water-soluble nanocrystals**

(62) Divisional of application: 01917351.7
(71) Applicant: L'UNIVERSITE DE REIMS CHAMPAGNE-ARDENNE, 51097 Reims cedex (FR); SCIENTIFIC DEVELOPMENT SERVICE STROISNABSERVICE, 113035 Moscow (RU)
(72) Inventor: Nabiev, Igor, 51100 Reims (FR); Sukhanova, Alyona, 51100 Reims (FR); Jardillier, Jean-Claude, 51100 Reims (FR); Sharapov, Oleg, 31011 Kharkov (UA); Artemyev, Mikhail, Minsk (BY); Baranov, Alexandre, Saint Petersbourg (RU)
(74) Representative: Laget, Jean-Loup

(57) **Abstract**

The invention concerns water-soluble semiconductor nanocrystal comprising semiconductor core and shell as well as a cap of one or more additional compounds comprising water-solubilization agents. In that the water-solubilization agent is a molecule of structural formula (R¹)ₐ-R²-{(R³)_{b}(R⁴)_{c}}_{d}.

## Description

The present invention relates to ultrasensitive non-isotopic water-soluble nanocrystals for use in non-isotopic detection systems especially as probes for biological applications wherein the probes are able of providing a detectable electron paramagnetic resonance (EPR) signal or a fluorescence signal or combination thereof in response to exposure to electromagnetic radiation.

Non-isotopic detection systems have become a preferred mode rather than the use of radioactive markers in scientific research and clinical diagnostics for the detection of biomolecules using various assays such as DNA sequencing, nucleic acid amplifications, immunohistochemistry, etc...

The semiconductor nanocrystals (quantum dots) are useful in biological applications as they can be rendered water-soluble, i.e. sufficiently soluble or suspendable in a aqueous-based solution such as in water or water-bases solutions or buffer solutions including those used in biological or molecular detection systems.

To this aim, WO-A-0027365 proposes a composition of functionalized nanocrystals comprising quantum dots capped with a capping compound comprising mercaptocarboxylic acid forming a first layer and a second layer comprising diaminocarboxylic acid linked to the capping compound and further layers including amino acid, or affinity ligand linked to the diaminocarboxylic acid.

In WO-A-00/58731, the nanoparticles are made water-soluble by linking with a water-soluble amino derivative of a polysaccharide.

WO-A-00/28088 and WO-A-00/28089 describe water-soluble nanocrystals of the type described in WO-A-0027365 having polynucleotide strands to form dendrimers or nucleobases that can be detected by fluorescence emission.

WO-A-00/17656 describes a water-soluble semiconductor nanocrystal comprising as solubilization agent SH(CH₂)ₙX wherein X is carboxylate or sulfonate.

WO-A-00/17655 describes a water-soluble semiconductor nanocrystal comprising as solubilization agent a molecule having an hydrophobic region and an hydrophilic group consisting of carboxylic acid, carboxylate, sulfonate, hydroxide, alkoxides, ammonium salts, phosphate, phosphonate, methacrylic acid, acrylic acid, hydrophilically derivatized styrene, or molecules having the formula (R¹)ₐ-R²-[(R³)_{b}(R⁴)_{c}]_{d} wherein R¹ is selected from the group consisting of heteroalkyl, heteroalkenyl, heteroalkynyl, -OR, -SR, -NHR, -NR'R", -N(O)HR, -N(O)R'R", -PHR, -PR'R", - P(NR'R")NR'R", -P(O) (NR'R")NR'R", -P(O) (OR')OR", - P(O)OR, -P(O)NR'R ", -P(O) (NR'R") NR'R", -P(O) (OR')OR", - P(O)OR, -P(O)NR'R", -P(S) (OR')OR", and -P(S)OR" wherein R, R' and R" are independently selected from the group consisting of H, a branched or unbranched alkyl, a branched or unbranched alkenyl, a branched or unbranched heteroalkyl, a branched or unbranched heteroalkenyl and a branched or unbranched heteroalkynyl with the proviso that when a is greater than 1, the R' groups can be same or different or can be linked to form a six-, seven-, eight-, nine- or ten-membered cycloalkyl, cycloalkenyl, heterocyclic, aryl, heteroaryl or a six- to thirty membered crown ether or heterocrown ether ; R² is selected from a bond, a branched or unbranched alkylene, a branched or unbranched heteroalkylene, cycloalkyl, cycloalkenyl, heterocyclic aryl and heteroaryl, R³ is selected from a branched or unbranched alkylene, a branched or unbranched alkenylene, a branched or unbranched heteroalkylene, a branche or unbranched hetereoalkenylene, cycloalkyl, cycloalkenyl, cycloalkynyl, heterocyclic aryl and heteroaryl, R⁴ is selected from the group consisting of an hydrogen, a carboxylate, a thiocarboxylate, an amine, an amide, an imine, an hydrazine, a sulfonate, a sulfoxide, a phosphate, a phosphonate, a phosphonium, an alcohol, a thiol, an ammonium, an alkyl ammonium, a nitrate, a sugar moiety and a five, six, seven, eight, nine, ten-membered cycloalkyl, cycloalkenyl, cycloalkynyl, heterocyclic aryl or heteroaryl, a being 1, 2, 3 or 4, b being 0, 1, 2 or 3, c being 0, 1, 2 or 3, d being 0, 1, 2 or 3.

WO-A-00/17642 describes a composition comprising fluorescent semiconductor nanocrystals associated to a compound, the spectral emission of the nanocrystals providing information about a biological state or event, the nanocrystals providing information about a biological state or event, the nanocrystals being water-soluble because of a ligand having at least a linking group linked to the nanocrystal and an hydrophilic group.

WO-A-00/29617 describes water-soluble luminescent quantum dot and biomolecular conjugate thereof for ultrasensitive non-isotopic detection in vitro and in vivo. The water-soluble luminescent quantum dot comprises a core consisting of a nanoparticle-sized semiconductor (CdS or CdSe), a cap consisting of a semiconductor differing from the one of the core (ZnS or CdS) and an hydrophilic attachment group consisting of any organic group that can be attached to the surface of the cap and renders the quantum dot water-soluble. The binding between the cap and the hydrophilic attachment group is realised via a sulphur atom.

The water-soluble luminescent semiconductor quantum dot can then be linked directly or indirectly with a biomolecule like a protein, a fragment of protein or a nucleic acid, via the hydrophilic attachment group.

The aim of the present invention is to propose a water-soluble nanocrystal prepared with novel solubilisation agents and the method of water-solubilisation of the nanocrystals. Also provided are water-soluble semiconductor nanocrystals probes for biological application wherein the probes are able of providing a detectable electron paramagnetic resonance (EPR) signal or a fluorescence signal or a combination thereof in response to exposure to electromagnetic radiation.

The water-soluble semiconductor nanocrystal according the invention comprises semiconductor core and shell as well as a cap of one or more additional compounds comprising water-solubilization agents, and is **characterized in that** the water-solubilization agent is chosen in the group consisting of :
- hydroxamates or derivatives of hydroxamic acid or a combination thereof,
- multidentate complexing agents or a combination thereof,
- a bilayer of a TOPO layer attached to the surface of the nanocrystal and an appropriate surfactant layer,
- a polymeric bead comprising carboxylic groups or amino groups or a combination thereof at the surface of the bead comprising one or more nanocrystals embedded within,
- a molecule of the structural formula (R¹)ₐ-R²-{(R³)_{b}(R⁴)_{c}}_{d.}

The core is a semiconductor nanoparticle. While any core of the IIA-VIB, IIIA-VB or IVA-VIB semiconductors can be used, the core must be such that a luminescent quantum dot results upon combination with a shell. The core is preferably CdSe or ZnSe.

The shell is a semiconductor that differs from the one of the core and bonds to the core and is preferably a IIA-VIB semiconductor of a band gap higher than the core band gap, preferably ZnS, CdS or ZnSe.

According to one embodiment of the invention, the water-solubilizing agent is hydroxamate or a derivative of hydroxamic acid or a combination thereof.

Accordingly, nanocrystal powder is dissolved in an aliphatic solvent and then mixed with a water solution of derivative of hydroxamic acid, the aliphatic layer containing the nanocrystals is extracted and the nanocrystals are precipitated by addition of an alcohol solvent, the precipitate being then dried to powder or dissolved in water.

The invention also provides that the multidentate complexing agent as water-solubilizing agent includes derivatives of ethylenediamine, diethylenetriamine, triethylenetetramine, tetraethylenepentaamine including multidentate acids, amines, thiols, phosphines, phosphates, phosphinoxides or a combination thereof.

Accordingly, nanocrystal powder is dissolved in an aliphatic solvent and then mixed with a water solution of multidentate complexing agents or a combination thereof, the aqueous layer containing the nanocrystals is extracted and the nanocrystals are precipitated by addition of an alcohol solvent, the precipitate being then dissolved in water.

According to the invention, the water-solubilisation agent can also be the molecule of structural formula (R¹)ₐ-R²-{(R³)_{b}(R⁴)_{c}}_{d} wherein :
R¹ is selected from the group consisting of heteroalkyl, heteroalkenyl, heteroalkynyl, -SR, -NHR, - NR₂, -N(O)HR, - N(O)R₂, -PHR, -PR₂, -P(NR₂), - P(O)R₂, -P(O) (NR₂)NR₂, -P(O) (OR)POR, -P(O)OR, -P(S) (OR)OR, -P(S)OR, - P(S)SH, -P(S)SR, -C(S)SH, -C(S)SR, wherein R is Li, Na, K ; Rb, Cs, NH₄, PH₄, R² is selected from a bond, a branched or unbranched alkylene, a branched or unbranched alkenylene, a branched or unbranched heteroalkylene, a branched or unbranched heteroalkenylene, cycloalkyl, cycloalkenyl, heterocyclic aryl and heteroaryl,
R³ is selected from a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic aryl and heteroaryl,
R⁴ is selected from the group consisting of hydrogen, a carboxylate, a thiocarboxylate, an amine, an amide, an imine, an hydrazine, a sulfonate, a sulfoxide, a phosphate, a phosphonate, a phosphonium, an alcohol, a thiol, an ammonium, an alkyl ammonium, a nitrate, a sugar moiety and a five, six, seven, eight, nine, then-membered cycloalkyl, cycloalkenyl, cycloalkynil, heterocyclic aryl or heteroaryl,
a being 1, 2, 3 or 4,
b being 0, 1, 2 or 3,
c being 0, 1, 2 or 3,
d being 0, 1, 2 or 3.

The invention also provides the process for water-solubilisation of the nanocrystals with TOPO (trioctylphhosphine oxide) and SDS. The nanocrystals are dissolved in tetrahydrofuran to which an appropriate surfactant molecules such as sodium dodecyl sulphate (SDS) is added, the mixture is stirred few hours, the solvent is evaporated at room temperature and the resulting powder is dissolved in water under ultrasonic treatment.

The invention has for further object the process for the preparation of polymeric water-soluble bead capped with carboxylic groups, comprising one or more nanocrystals embedded within the polymeric matrix **characterized in that** nanocrystals are dissolved in a mixture of appropriate monomers, a radical initiator is added, the emulsion is prepared in water, containing surfactant, and polymerised at 60-90°C within few hours under vigorous stirring, the polymeric beads being isolated by centrifugation and re-dissolved in water.

Preferably, the nanocrystal according the invention is doped with paramagnetic ions such as Mn² + and other transition or rare-earth ions.

To this purpose, the process for the fabrication of doped nanocrystals with paramagnetic ions is **characterised in that** TOPO is dried and degassed at 180°C for approximately 1 hour under argon atmosphere, a solution of elemental semiconductors and paramagnetic ions in TOP (tri-n-phosphine) is added to this melt at 350°C, the reaction mixture is then cooled to 300°C and a second solution of semiconductors is added and the reaction mixture is then cooled to 105°C, finally the semiconductor core and shell doped with paramagnetic ions are precipitated and washed with methanol and dissolved with hexane, heptane or tetrahydrofuran or dried to powder.

The invention also provides a water-soluble nanocrystal probe comprising a semiconductor nanocrystal, a water-solubilisation agent, a linking agent and an affinity moiety which is able to bound specifically to detectable biological substances, **characterized in that** the water-soluble nanocyrstal is one according to the invention.

According to one embodiment, the linking agent is able to connect the water-soluble nanocrystal or the polymeric bead comprising one or more nanocrystals embedded within with the affinity molecules including peptides, proteins, monoclonal or polyclonal antibodies and their functional fragments, oligonucleotides, RNA, single and double stranded DNA, RNA-DNA hymeric molecules, DNA triplexes and multiplexes ans carbohydrates which are able to bound specifically to detectable biological substances, e.g. proteins, oligonucleotides, DNA, RNA, viruses, bacteria, cells, including living cells and tissues.

Preferably, the water-solubilisation agent is also the linking agent.

The nanocrystals probes consisting of polymeric beads should have the same field of application as widely used known Nanofluorospheres comprising organic dyes molecules embedded in the polymeric beads. Advantages of the polymeric beads with the embedded nanocyrstals doped or undoped with the paramagnetic ions, compared with the Nanofluorospheres are as follows :
- 100-fold improved stability of nanocrystals against photobleaching as compared to organic dyes ;
- possibility of multicolour labelling using the nanocrystals of different diameters and of different core and shell ;
- possibility of using probes comprising nanocrystals doped with the paramagnetic ions as fluorescence or magnetic resonance probes or as a combination thereof.

The invention further comprises the method of detection of biological substances by registration of EPR signal from paramagnetic ions incorporated into the nanocrystals or of fluorescence signal from the nanocrystal or a combination thereof or by the construction of the images of biological substances by fluorescence or EPR microscopy or by a combination thereof.

The biological imaging is provided by scanning the object by following ways:
- via detection of special distribution of the nanocrystal flurorescence by fluorescence microscopy,
- via detection of the EPR signal with high spatial resolution by technique analogous tot the scanning tunnel microscopy with a ferromagnetic crystal needle producing the local magnetic field (Hochi A, Furusawa M, Ikeya M, Applications of microwave scanning ESR microscope : human tooth with metal. Appl Radiat Isot 1993 Jan-Feb;44 (1-2) : 401-405) or by cavity technique with the use of a narrow aperture for penetration of the microwave electromagnetic field in the object ;
- via simultaneous detection of the EPR signal with high spatial resolution and of the fluorescence signal with high spatial resolution.

The invention will be further described in more details in reference to the following examples and to the annexed drawing wherein :
Figure 1 is a schematic illustration of the doped nanocrystals of the invention ;
Figure 2a is a schematic illustration of the water-solubilisation of the nanonocrystals with hydroxamates of amino acids or with compounds prossessing hydroxamic acid functional group.
Figure 2b is a schematic illustration of the water-solubilisation of the nanocrystals with the multidentate complexing agent.
Figure 2c is a schematic illustration of the water-solubilisation of the nanocrystals with the bilayer of TOPO and sodium dodecyl sulphate (SDS).
Figure 2d is a schematic illustration of the water-solubilisation of the nanocrystals by incorporation of one or more TOPO-capped nanocrystals into polymeric bead.
Figure 3a is a schematic illustration of anchoring of the affinity molecules to the water-soluble nanocrystal capped with the hydroxamate of amino acid or with compounds possessing hydroxamic acid functional group.
Figure 3b is a schematic illustration of anchoring of the affinity molecules to the water-soluble nanocrystal capped with the multidentate complexing agent.
Figure 3c is a schematic illustration of anchoring of the affinity molecules to the water-soluble nanocrystal capped with the bilayer of TOPO and SDS.
Figure 3d is a schematic illustration of anchoring of the affinity molecules to the polymeric beads activated with the carboxylic groups and comprising TOPO-capped nanocrystals.

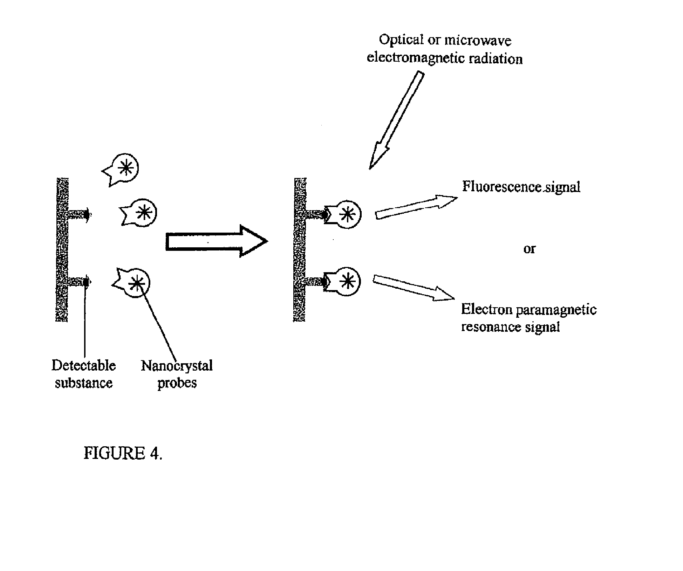

Figure 4 is a schematic representation of the use of water-soluble nanocrystral probe of the invention linked with the affinity molecule for ultrasensitive non-isotopic detection and analysis of a detectable substance in a biological material by fluorescence or by electron paramagnetic resonance (EPR) technique or by the combination thereof.
Figures 5a, 5b, 5c are examples of applications of the water-soluble nanocrystals probes according the invention for detection of biological objects.

The following examples serve to illustrate the present invention and are not intended to limit the scope of the invention.

### Example 1: fabrication of nanocrystals of the invention doped with Mn²⁺ ions.

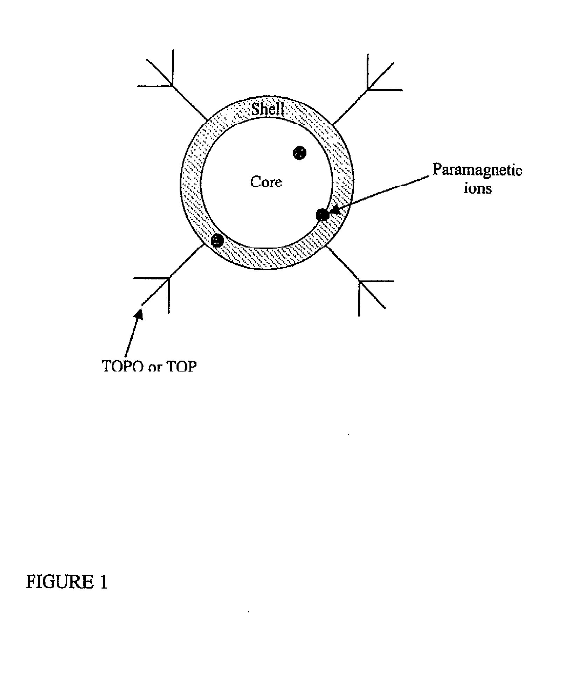

12g of TOPO placed in a three-neck flask are dried and degassed at 180°C for approximately 1 hour under the argon atmosphere. The solution of elemental selenium, dimethylcadmium and bis(phenyl selenide manganese) in 1,2 ml TOP was added under vigorous stirring to this melt at 350°C. The relative molar concentration of Cd/Se/Mn in TOP is 1,0/0,9/<0,05. The cadmium content in TOP is within the range of 0,1 mmol. After addition of TOP solution, the reaction mixture is cooled to 300°C and the second solution of dimethylzinc and of hexamethyldisilthiane in 2 ml TOP was added. The relative molar concentration of Cd/Zn in final mixture is about 1/4. After adding of the second TOP solution, the reaction mixture is cooled down to 105°C and allowed to stir another 1,5 hours. Finally, CdSe nanocrystals doped with Mn ions and capped with ZnS shell are precipitated with methanol, washed with methanol and dissolved in hexane, heptane, tetrahydrofurane or dried to powder. (figure 1)

### Example 2: water-solubilisation of nanocrystal capped with the glycine hydroxamate and the binding thereof to affinity biological molecules.

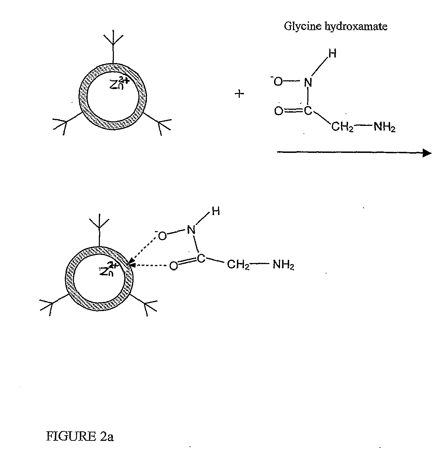

Few milligrams of nanocrystals powder are dissolved in heptane and mixed with water solution of glycine hydroxamate (0,1-0,001 M) under vigorous stirring. Approximately in one hour, the nanocrystals are extracted from the aliphatic layer due to bonding of the hydroxamate to the Zn-atoms on the nanocrystal surface. The aliphatic layer is further removed and nanocrystals are precipated by adding methanol, ethanol, propanol or acetone. The precipitate is dried to powder, or dissolved in fresh portion of water for further manipulations (figure 2a).

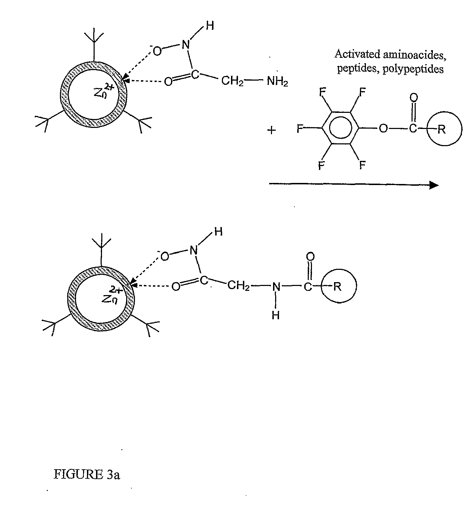

The binding of such water-soluble nanocrystals with biomolecules of interest such as amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc. may be further proceeded as described in Bioconjugate techniques, 1996, E.Hermanson, Academic Press, using the free NH₂-group of the nanocrystal-attached hydroxamate as an active linker group as presented in figure 3a wherein the nanocrystal probe reacts with activated aminoacids, peptides or polypeptides and R is amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc.

### Example 3: Fabrication of water-soluble nanocrystal capped with the diethylenetriaminepentakis (methylphosphonic acid) and the process of binding therof to affinity biological molecules.

The solubilisation of nancrystals with multidentate ligands of ethylenediamine groups is conducted via the procedure described in example 2 for glycine hydroxamate.

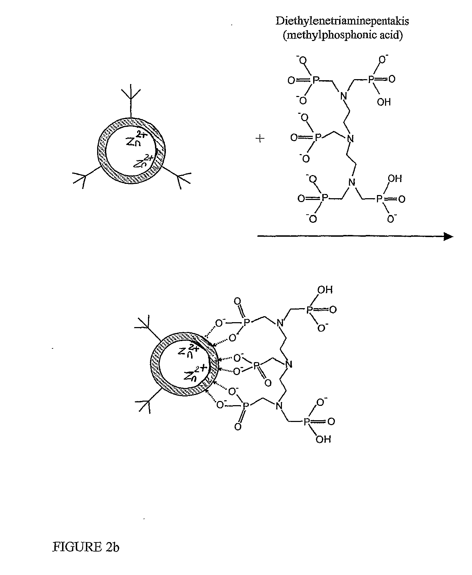

Briefly, few milligrams of nanocrystals are dissolved in heptanes and mixed with water solution of diethylenetriaminepentakis(methylphosphonic acid) under vigorous stirring. In one hour, aliphatic layer is removed and nanocrystals are precipitated by alcohols or acetone and re-dissolved in fresh portion of water. (figure 2b)

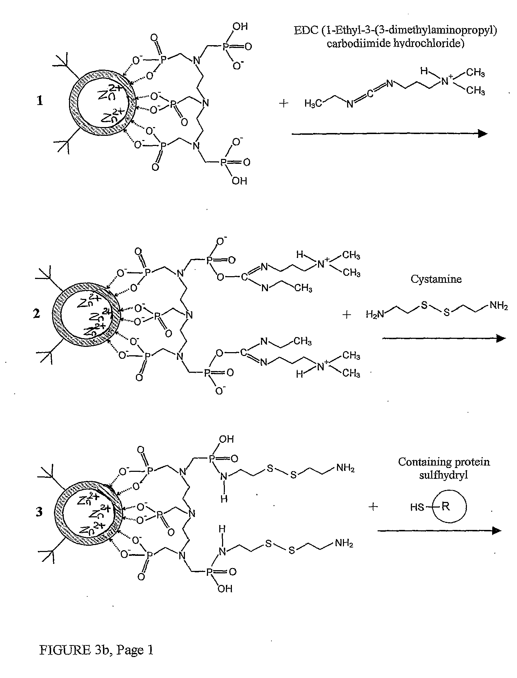

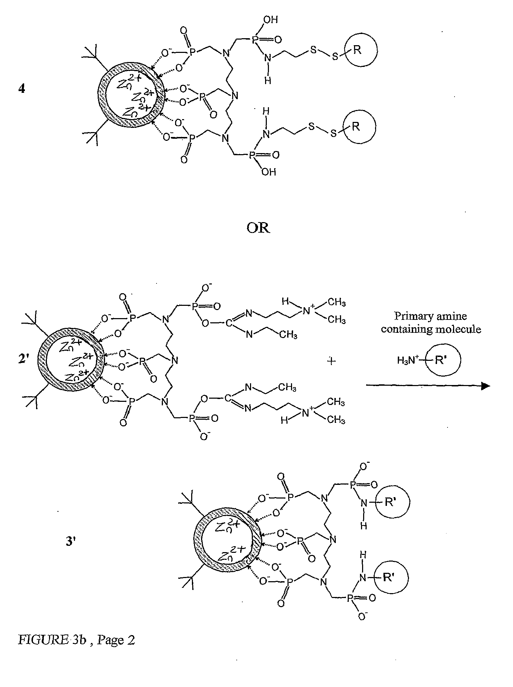

The binding of such water-soluble nanocrystals with biological molecules of the interest such as amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc. may be further proceeded as described in Bioconjugate techniques, 1996, Ed. Hermanson, Academic Press, as presented in figure 3B wherein respectively the nanocrystal probe (1) reacts with 1-ethyl-3-(3-dimethylaminopropyl)carbodiimide hydrochloride (EDC) to form (2), then with cystamine as linking agent to form (3), further with a containing protein sulfhydryl to form (4), or with EDC to form (2'), then with a primary amine containing molecule to form (3') wherein R and R' are the same as R in example 2.

### Example 4: fabrication of water-soluble nanocrystals by treatment thereof with the sodium dodecyl sulphate and formation of bilayer around of nanocrystal shell, and the process of binding of linking agent (EDAC), which was ever bounded with the affinity molecule and creation of conjugate with the biomolecule.

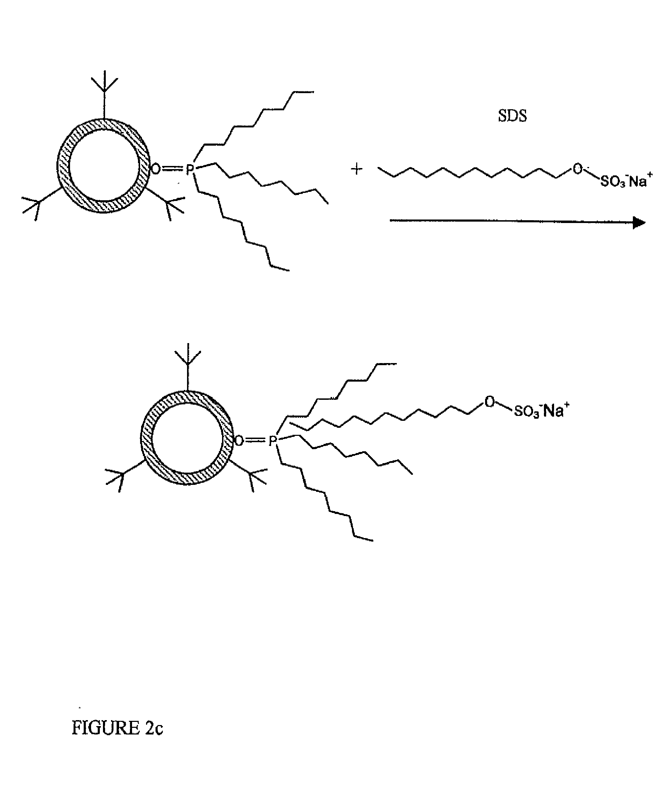

Few milligrams of nanocrystals are dissolved in tetrahydrofurane to which sodium dodecylsulfate (SDS) is added. The relative molar concentration of nanocrystal/SDS/ is 1/1 to 1/1000. The mixture is stirred few hours and solvent is evaporated at room temperature. The resulting powder is dissolved in water under ultrasonic treatment (figure 2c).

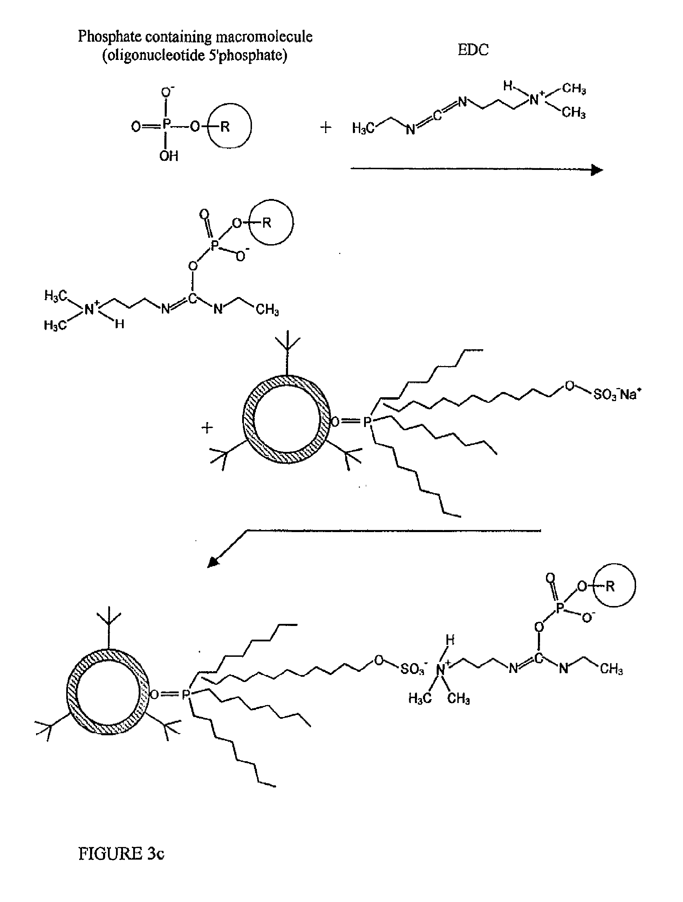

The binding of such water-soluble nanocrystals with biological molecules of interest such as amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc.., may be further proceeded as described in Bioconjugate techniques, 1996, Ed.Hermanson, Academic Press, as presented in figure 3c wherein R is same as in example 2.

### Example 5: Fabrication of polymeric water-soluble bead capped with the carboxyl groups, comprising one or more nanocrystals embedded within the polymeric matrix ; binding of linking agent and affinity molecules to the bead and creation of bioconjugate.

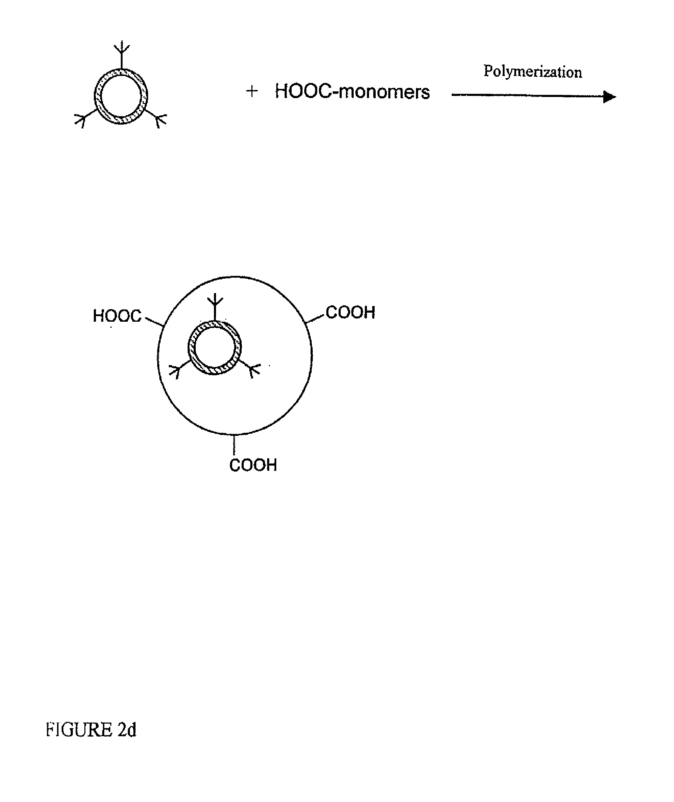

Few milligrams of nanocrystals are dissolved in a mixture of methylmethacrylate/hexylmethacrylate/methacrylic acid or methylmethacrylate/decylmethacrylate/methacrylic acid at various molar concentrations : 100/0/1 to 0/100/1. The radical initiator AIBN or its derivative is added to approximately 0,1-5%(w/w) and this solution is mixed with water to which 0,0001-0,1%(w/w) of SDS or hexadecyltrimethylammonium bromide was added. Under vigorous stirring, the emulsion is polymerized at 60-90°C within few hours. Finally, polymeric beads are isolated by centrifugation and re-dissolved in fresh portion of water (figure 2d).

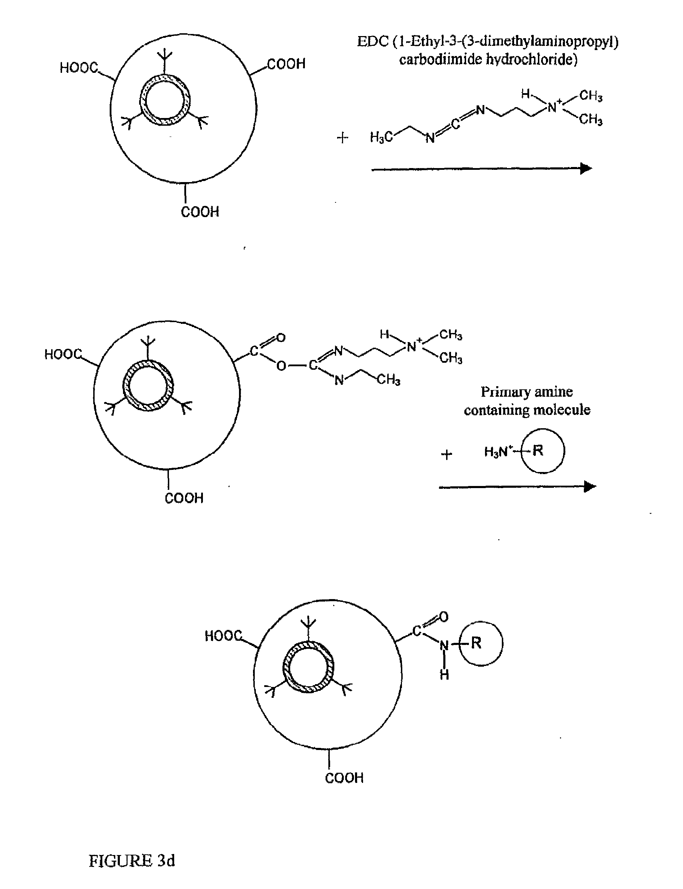

The binding of such water-soluble nanocrystals with biological molecules of interest such as amino acids, peptides, proteins, monoclonal or polyclonal antibodies and their functional fragments, oligonucleotides, RNA, single or double-stranded DNA, RNA-DNA hymeric molecules, DNA triplexes and multiplexes and carbohydrates, eg sialic acids, etc. may be further proceeded as described in Bioconjugate techniques, 1996, Ed.Hermanson, Academic Press, as presented in figure 3d wherein R is the same as in example 2.

### Example 6: use of the water-soluble doped or undoped nanocrystal probes for detection of biological substances and preparation of samples containing nanocrystal probes of the invention and recording of fluorescence image o biological object (living cell or in vitro immunological assays).

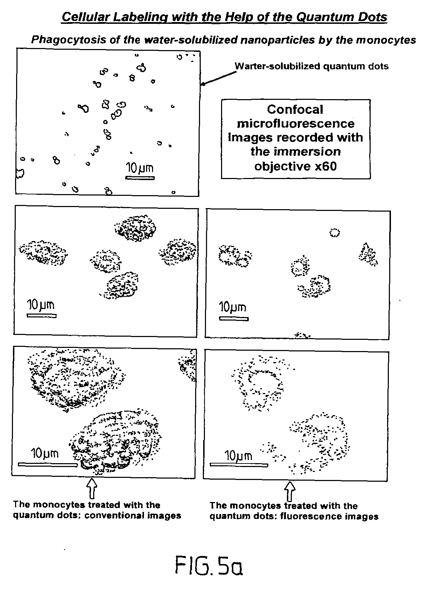

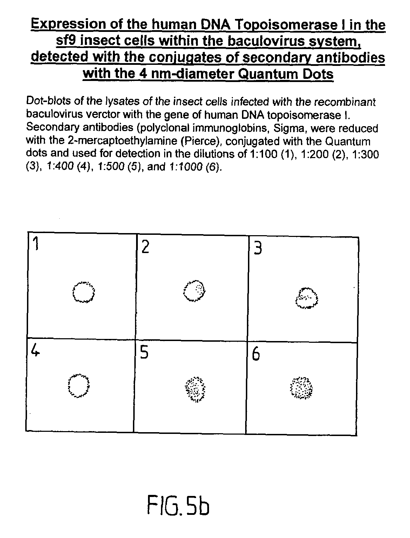

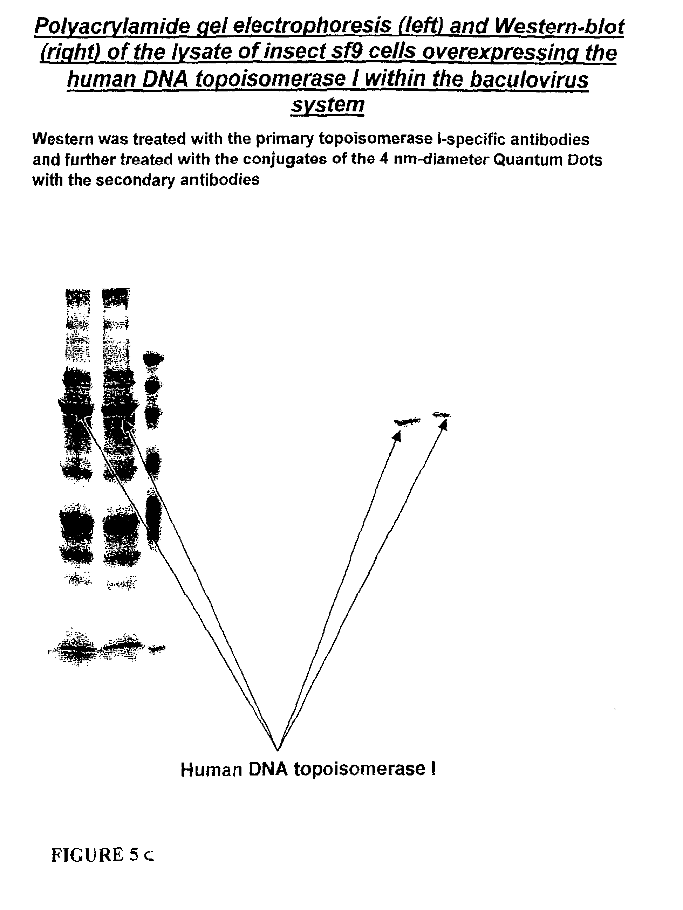

The figures 5a, 5b and 5c show examples of application of the water-soluble nanocrystals of the invention for fluorescence ex-vivo imaging of biological object (monocytes treated with the water-solubilized nanocrystals) and the results of application of the antibodies-nanocrystals conjugates in the Western blot and dot-blot immunoassays for ultrasensitive detection of biological substances.

The samples were prepared using conventional techniques and the experimental conditions are presented in the figures.

The cellular labelling with the help of the quantum dots according to the invention is illustrated in figure 5a.

As seen in figure 5c, antibodies-nanocrystals conjugates are used in the Western blot. The Western blot was treated with the primary topoisomerase I-specific antibodies and further treated with the conjugates of the 4nm-diameter quantum dots with the secondary antibodies.

As seen in figure 5b, antibodies-nanocrystals conjugates are used in dot-blot immunoassays for ultrasensitive detection of biological substances.

## Claims

1. Water-soluble semiconductor nanocrystal comprising semiconductor core and shell as well as a cap of one or more additional compounds comprising water-solubilization agents,
**characterized in that** the water-solubilization agent is a molecule of structural formula (R¹)ₐ-R²-{ (R³) _{b}(R⁴)_{c}}_{d}.

2. Water-soluble semiconductor nanocrystal according to the claim 1, **characterized in that** the molecule of structural formula (R¹)ₐ-R²-{(R³)_{b}(R⁴)_{c}}_{d} is such as:
R¹ is selected from the group consisting of heteroalkyl, heteroalkenyl, heteroalkynyl, -SR, -NHR, -NR₂, -N(O)HR, -N(O)R₂, -PHR, -PR₂, -P(NR₂), - P(O)R₂, -P(O)(NR₂)NR₂, -P(O)(OR)POR, -P(O)OR, -P(S)(OR)OR, -P(S)OR, - P(S)SH, -P(S)SR, -C(S)SH, -C(S)SR wherein R is Li, Na, K; Rb, Cs, NH₄, PH₄,
R² is selected from a bond, branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic aryl and heteroaryl,
R³ is selected from a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic aryl and heteroaryl,
R⁴ is selected from the group consisting of hydrogen, a carboxylate, a thiocarboxylate, an amine, an amide, an imine, an hydrazine, a sulfonate, a sulfoxide, a phosphate, a phosphonate, a phosphonium, an alcohol, a thiol, an ammonium, an alkyl ammonium, a nitrate, a sugar moiety and a five, six, seven, eight, nine, ten-membered cycloalkyl, cycloakenyl, cycloalkynil, heterocyclic, aryl or heteroaryl,
a being 1, 2, 3 or 4,
b being 0, 1, 2 or 3,
c being 0, 1, 2 or 3,
d being 0, 1, 2 or 3.

3. Water-soluble semiconductor nanocrystal according to the claim 1, **characterised in that** the nanocrystal is doped with paramagnetic ions.

4. Water-soluble nanocrystal probe comprising a water soluble semiconductor nanocrystal according to claim 1, a water-solubilisation agent, a linking agent and an affinity moiety which is able to bound specifically to detectable biological substances.

5. Probe according to the claim 4,
**characterized in that** the linking agent is able to connect the water-soluble nanocrystal or the polymeric bead comprising one or more nanocrystales embedded within, with the affinity molecules including peptides, proteins, monoclonal or polyclonal antibodies and their functional fragments, oligonucleotides, RNA, single and double stranded DNA, RNA-DNA hymeric molecules, DNA triplexes and multiplexes and carbohydrates which are able to bound specifically to detectable biological substances.

6. Probe according to the claims 4 and 5,
**characterized in that** the water-solubilisation agent is also the linking agent.

7. Process for the fabrication of doped nanocrystals with paramagnetic ions according to the claim 3,
**characterised in that:**
a TOPO is dried and degassed at 180°C for approximately 1 hour under argon atmosphere,
a solution of elemental semiconductors and the paramagnetic ions in TOP to this melt at 350°C is added
the reaction mixture is then cooled to 300°C and
a second solution of semiconductors is added and the reaction mixture is then cooled to 105°C,
finally the semiconductor core and shell doped with paramagnetic ions are precipitated and washed with methanol and dissolved with hexane, heptane or tetrahydrofuran or dried to powder.

8. Method of detection of biological substances by registration of EPR signal from paramagnetic ions incorporated into the nanocrystals according to the claim 3.

9. Method of detection of biological substances by registration of fluorescence signal from the nanocrystal according to anyone of the claims 1 or 3.

10. Method of detection of biological substances by registration of a combination of fluorescence signal and EPR signal from paramagnetic ions incorporated into the nanocrystals according to claim 3.

11. Method of detection of biological substances by the construction of images of biological substances by fluorescence or EPR microscopy or by a combination thereof from the nanocrystals according to claims 1 or 3.
